# EUROPÄISCHE PATENTSCHRIFT

(11) **EP 1 575 457 B1**
(45) Veröffentlichungstag und Bekanntmachung des Hinweises auf die Patenterteilung: **07.05.2008**
(21) Anmeldenummer: 02782618.9
(22) Anmeldetag: 17.12.2002
(51) Int. Cl.: A61F 2/44

(54) **ZWISCHENWIRBELIMPLANTAT**
INTERVERTEBRAL IMPLANT
IMPLANT INTERVERTEBRAL

(43) Veröffentlichungstag der Anmeldung: 21.09.2005
(73) Patentinhaber: Synthes GmbH, 4436 Oberdorf (CH)
(72) Erfinder: AEBI, Max, CH-3011 Bern (CH); BURKARD, Dominique, CH-5014 Gretzenbach (CH); FRIGG, Robert, CH-2544 Bettlach (CH); LECHMANN, Beat, CH-2544 Bettlach (CH); MATHYS, Robert, Jr., CH-2544 Bettlach (CH); PAVLOV, Paul, NL-6523 NA Nijmegen (NL)
(74) Vertreter: Lusuardi, Werther
(86) Internationale Anmeldenummer: PCT/CH2002/000707
(87) Internationale Veröffentlichungsnummer: WO 2004/054478

(56) Entgegenhaltungen:
- WO-A-01/56513
- US-A- 4 759 766
- US-A- 5 556 431

## Beschreibung

Die Erfindung bezieht sich auf ein Zwischenwirbelimplantat gemäss dem Oberbegriff des Patentanspruchs 1.

Nach Entfernung einer beschädigten, natürlichen Bandscheibe oder eines beschädigten Nukleus pulposus einer Bandscheibe werden Implantate oder Prothesen in den Zwischenwirbelraum zweier benachbarter Wirbelkörper eingebracht. Dabei entsteht das Ziel, wieder möglichst natürliche Zustände herbeizuführen, d.h. insbesondere die ursprüngliche Bandscheibenhöhe und damit den ursprünglichen Abstand zwischen den beiden benachbarten Wirbelkörpern wiederherzustellen. Ferner sollen Bewegungen der benachbarten Wirbelkörper relativ zueinander möglichst ohne Behinderung in ihrer natürlichen Art ausführbar sein. Hierzu ist die Erhaltung der Bewegungsmöglichkeiten bei einer Vorwärts/Rückwärtsneigung, d.h. Flexion und Extension der Wirbelkörper sowie bei einer lateralen Beugung der Wirbelkörper innerhalb der natürlichen Grenzen wesentlich. Die natürlichen Bänder und Muskeln entlang der Wirbelsäule werden im wesentlichen intakt gelassen, so dass diese die Bewegungen eines mechanischen Bandscheibenersatzes weiter stabilisieren.

Eine gattungsgemässe Bandscheibenendoprothese ist aus der US 4,759,766 BÜTTNER bekannt. Diese bekannte Bandscheibenendoprothese besteht im wesentlichen aus zwei symmetrischen Abschlussplatten mit gegeneinander gerichteten konkaven Gleitflächen und je einer aussenstehenden Oberfläche zur Anlage an die Grundplatte, respektive die Deckplatte der angrenzenden Wirbelkörper und einem zwischen den Abschlussplatten positionierten Distanzstück mit zu den konkaven Gleitflächen an den Abschlussplatten komplementär ausgestalteten konvexen Gleitflächen. Die Gleitflächen sind in einer Ausführungsform als Teilflächen einer Zylindermantelfläche ausgebildet, wobei die an den beiden Abschlussplatten angeordneten Gleitflächen komplementär zu je einer der angrenzenden Gleitflächen am Distanzstück ausgestaltet sind und je zwei komplementäre Gleitflächen die aufeinander verschiebbaren Artikulationsflächen eines um eine Drehachse rotierbaren Gelenkteiles bilden. Das Gelenk umfasst ein oberes und ein unteres Gelenkteil mit je einer Drehachse. Die beiden Drehachsen sind um 90° zueinander versetzt. Nachteilig an dieser bekannten Bandscheibenendoprothese ist, dass
a) den durch die natürliche Bandscheibe übertragbaren überlagerten Schwenkbewegungen insbesondere bei anterior-posterior und lateraler Flexion, welche bei der natürlichen Bandscheibe unabhängig voneinander sind, durch die Ausgestaltung einer Bandscheibenendoprothese mit nur einem Drehzentrum nicht Rechnung getragen wird;
b) durch Scherbewegungen, insbesondere bei Translation in anterior-posteriorer Richtung das Wirbelgelenk (Facettengelenk) belastet wird, wodurch für den Patienten Schmerzen verursacht werden können;
c) nachteilige Reibungskräfte bei zwei aufeinander gleitbaren, artikulierenden Flächen entstehen. Ferner sind an den Flächen Verschleiss, d.h. unter anderem auch Abrieb sowie Widerstand bei der Bewegung der Gelenkteile die Folge. Zudem besteht das Risiko des "Stick-Slip" Effektes;
d) ein mechanischer Bandscheibenersatz die weitere Degeneration der betroffenen Bewegungssegmente kaum aufhalten kann. Das Wiederherstellen der ursprünglichen Bewegungsverhältnisse reduziert den Schmerz wesentlich und der Patient gewinnt an Lebensqualität. Bei neuem Auftreten von Schmerz muss jedoch eine Revision der Versorgung in Angriff genommen werden. Dabei wird üblicherweise eine Bandscheibenprothese nach herkömmlicher Bauart komplett entfernt und das Bewegungssegment versteift. Diese Operation belastet den Patienten ausserordentlich; und
e) der Form der Kontaktflächen zu den benachbarten Wirbelkörpern in der Regel nicht Rechnung getragen wird. Bandscheibenersatzimplantate herkömmlicher Bauart haben plane (flache) Kontaktflächen, welche oft noch mit kielartigen Erhebungen ergänzt sind.

Hier will die Erfindung Abhilfe schaffen. Der Erfindung liegt die Aufgabe zugrunde, ein Zwischenwirbelimplantat zu schaffen, welches nur Drehbewegungen um bestimmte Drehachsen gestattet und keine Translationsbewegungen der Wirbelkörper zulässt.

Die Erfindung löst die gestellte Aufgabe mit einem Zwischenwirbelimplantat, welches die Merkmale des Anspruchs 1 aufweist.

Die durch die Erfindung erreichten Vorteile sind im wesentlichen darin zu sehen, dass dank des erfindungsgemässen Zwischenwirbelimplantates
- die Schwenkbewegungen in anterior-posteriorer Richtung und nach lateral sind unabhängig voneinander;
- Translationsbewegungen der Wirbelkörper sind ausgeschlossen, wodurch die Facettengelenke geschont werden können;
- die Drehachsen die anatomischen Gegebenheiten berücksichtigen.

Im erfindungsgemässen Zwischenwirbelimplantat sind die Gleitflächen als Teilflächen von Kreiszylindermantelflächen ausgestaltet. Anstelle von Teilflächen aus Kreiszylindermantelflächen sind auch Teilflächen aus anderen rotationssymmetrischen Zylindermantelflächen, beispielsweise Kegelmantelflächen möglich.

Im Zwischenwirbelimplantat ermöglichen die Mittel eine temporäre Blockierung der Beweglichkeit der beiden Teile um das Gelenk. Dadurch ist der Vorteil erreichbar, dass mittels eines minimal invasiven Eingriffes das im Zwischenwirbelraum integrierte Gelenk blockierbar ist. Dies ist besonders vorteilhaft in Fällen bei denen post-operativ Schmerzen auftreten, d.h. wo die Degeneration des betroffenen Wirbelsäulensegmentes weitergeht und der Chirurg eine Fusion der betroffenen Wirbel in Betracht zieht. Vorzugsweise sind die Mittel an den beiden ventralen Seitenflächen der beiden Teile anbringbar. Durch dieses spätere, sekundäre Blockieren der Bewegbarkeit der beiden Teile um das Gelenk wird das Zwischenwirbelimplantat versteift und in ein Arthrodesenimplantat (Fusions-Käfig) übergeführt.

In einer Ausführungsform des erfindungsgemässen Zwischenwirbelimplantates umfasst beispielsweise das untere Gelenkteil mindestens eine bezüglich der ersten Drehachse rotationssymmetrische, untere konkave Gleitfläche und das mittlere Gelenkteil mindestens eine zur unteren konkaven Gleitfläche komplementäre, untere konvexe Gleitfläche. Das obere Gelenkteil umfasst mindestens eine bezüglich der zweiten Drehachse rotationssymmetrische, obere konvexe Gleitfläche und das mittlere Gelenkteil mindestens eine zur oberen konvexen Gleitfläche komplementäre, obere konkave Gleitfläche. Eine Umkehrung der zwei Gleitflächenpaare, so dass das obere Gelenkteil mindestens eine bezüglich der ersten Drehachse rotationssymmetrische, obere konkave Gleitfläche umfasst und das mittlere Gelenkteil mindestens eine zur oberen konkaven Gleitfläche komplementäre, obere konvexe Gleitfläche umfasst, ist auch möglich. In diesem Falle gilt die Umkehrung auch für das untere Gelenkteil, welche dann mindestens eine bezüglich der zweiten Drehachse rotationssymmetrische, untere konvexe Gleitfläche umfasst, während hier das mittlere Gelenkteil mindestens eine zur unteren konvexen Gleitfläche komplementäre, untere konkave Gleitfläche umfasst. Durch die Ausgestaltung des Gelenkes derart, dass das mittlere Gelenkteil eine konvexe und bezüglich der Zentralachse axial gegenüberliegend, eine konkave Gleitfläche aufweist und die aussenstehenden Gelenkteile komplementär ausgestaltet sind, ist eine minimale Bauhöhe des Zwischenwirbelimplantates erreichbar.

Wegen der unterschiedlichen Positionen der natürlichen Drehachsen in den entlang der Wirbelsäule verschiedenen Bandscheibenräumen kann die Anordnung der Drehachsen windschief oder sich schneidend sein.

Als Materialpaarungen für die mit Gleitflächen ausgestatteten Teile des Zwischenwirbelimplantates eignen sich im wesentlichen Metall-Metall, Metall-Keramik oder Metall-Kunststoff Paarungen. Als Metall sind eisenhaltige oder nichteisenhaltige Metallegierungen bevorzugt, während als Keramikmaterialien vorzugsweise AlOₙ und ZrOₙ einsetzbar sind. Von den für Implantate einsetzbaren Kunststoffen werden vorzugsweise hochmolekulare Thermoplaste eingesetzt, allerdings lässt sich auch PEEK verwenden.

In einer anderen Ausführungsform des erfindungsgemässen Zwischenwirbelimplantates sind von den ventralen Seitenflächen her Mittel an den beiden Teilen anbringbar, wodurch die beiden Teile ventral auf einer bestimmten Distanz relativ zueinander gehalten werden können. Dadurch ist der Vorteil erreichbar, dass die beiden Teile zur Einführung in den Zwischenwirbelraum in eine Position mit fest gehaltener Höhe bringbar sind und nach der Einführung in den Zwischenwirbelraum um das Gelenk bewegbar und an die Grund- respektive Deckplatte der angrenzenden Wirbelkörper zur Anlage bringbar sind.

In wiederum einer weiteren Ausführungsform des erfindungsgemässen Zwischenwirbelimplantates umfassen die Mittel einen Einsatz, welche in je eine Vertiefung an den einander gegenüberliegenden Oberflächen des oberen und unteren Teiles einsetzbar ist. Vorzugsweise sind die Vertiefungen als Schwalbenschwanzführungen ausgestaltet, welche an den ventralen Seitenflächen offen sind, so dass die zu den Schwalbenschwanzführungen komplementär ausgestalteten Enden des Einsatzes von ventral in die Schwalbenschwanzführungen eingeschoben werden können. Dadurch ist der Vorteil erzielbar, dass durch das Einführen des Einsatzes die Bewegbarkeit der beiden Teile um das Gelenk blockierbar ist. Die Starrheit der Blockierung lässt sich erhöhen, wenn die Schwalbenschwanzführungen so ausgestaltet sind, dass sie sich gegen die Zentralachse des Zwischenwirbelimplantates verjüngen, so dass der Einsatz zusätzlich in den Schwalbenschwanzführungen verkeilbar ist.

In wiederum einer anderen Ausführungsform des erfindungsgemässen Zwischenwirbelimplantates sind die beiden Teile mit Bohrungen zur Aufnahme von Knochenfixationsmittel, insbesondere von Knochenschrauben versehen, wobei die Bohrungen Längsachsen aufweisen, welche schräg zur Zentralachse stehen. Vorzugsweise durchdringen je zwei Bohrungen eines der beiden Teile von der ventralen Seitenfläche zur Appositionsfläche. Dabei können die Längsachsen, falls nur eine axiale Fixierung des Zwischenwirbelimplantates vorgesehen ist, nur von lateral betrachtet schräg zur Zentralachse stehen, oder falls eine winkelstabile Fixierung des Zwischenwirbelimplantates vorgesehen ist, auch von ventral betrachtet von den inneren Oberflächen der beiden Teile gegen die Appositionsflächen divergieren.

In einer weiteren Ausführungsform des erfindungsgemässen Zwischenwirbelimplantates sind die Bohrungen zur Aufnahme der Knochenfixationsmittel mit Innengewinden versehen, wodurch sich eine zusätzliche, rigide Fixierung der Knochenfixationsmittel in den beiden Teilen erreichen lässt. Vorzugsweise sind die Bohrungen konisch ausgestaltet, so dass durch die konischen Gewindeverbindungen zwischen den Innengewinden und den Aussengewinden an den Köpfen der Knochenfixationsmittel eine verstärkte Fixierung der Knochenfixationsmittel an jedem der beiden Teile erreichbar ist.

Das Verfahren zum Einführen eines erfindungsgemäßen Zwischenwirbelimplantates dient im wesentlichen dem Ersatz einer defekten, natürlichen Bandscheibe durch ein Zwischenwirbelimplantat und stellt keinen Teil der Erfindung dar. Es umfasst die Schritte:
A) blockieren des oder der Gelenke eines Zwischenwirbelimplantates mittels dafür vorgesehener Mittel in einer bestimmten Position des oder der Gelenke;
B) einführen des Zwischenwirbelimplantates in den zu behandelnden Zwischenwirbelraum;
C) lösen und entfernen der zur Blockierung des oder der Gelenke in das Zwischenwirbelimplantat eingesetzten Mittel. Durch die Blockierung des Gelenkes ist der Vorteil erreichbar, dass die beweglichen Teile mit den aussenstehenden Appositionsflächen einfacher in den zu behandelnden Zwischenwirbelraum einführbar sind.

In einer weiteren Anwendung des Verfahren umfasst dieses das nachträgliche Blockieren des oder der Gelenke am implantierten Zwischenwirbelimplantat mittels der zur Blockierung des oder der Gelenke vorgesehenen Mittel. Dadurch ist der Vorteil erreichbar, dass bei einem Auftreten von post-operativen Schmerzen für den Patienten oder bei einer weiteren Degeneration des betroffenen Bewegungssegmentes das oder die Gelenke am Zwischenwirbelimplantat postoperativ durch Einsetzen der dazu vorgesehenen Mittel blockierbar sind. Diese nachträgliche Blockierung ist mit einem minimal-invasiven, vorzugsweise einem lapraskopischen Eingriff möglich. Das Zwischenwirbelimplantat übernimmt dann die Aufgabe eines Käfigs, so dass das betroffene Bewegungssegment der Wirbelsäule versteift werden kann.

Die Erfindung und Weiterbildungen der Erfindung werden im folgenden anhand der teilweise schematischen Darstellungen mehrerer Ausführungsbeispiele noch näher erläutert.

Es zeigen:
Fig. 1 eine Explosionsdarstellung einer Ausführungsform des erfindungsgemässen Zwischenwirbelimplantates;
Fig. 2 eine perspektivische Ansicht der in Fig. 1 dargestellten Ausführungsform des erfindungsgemässen Zwischenwirbelimplantates in zusammengesetztem Zustand;
Fig. 3 eine Ansicht von lateral auf eine weitere Ausführungsform des erfindungsgemässen Zwischenwirbelimplantates; und
Fig. 4 eine perspektivische Ansicht von ventral der Ausführungsform nach Fig. 3.

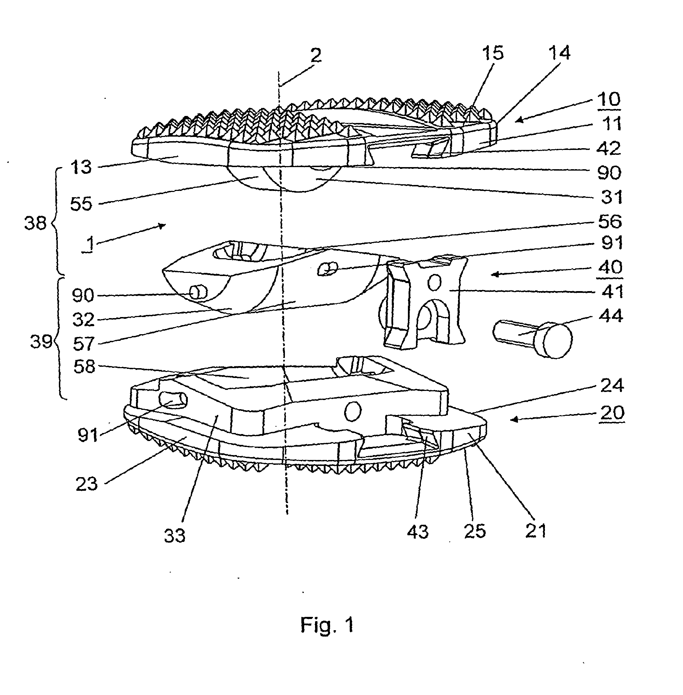

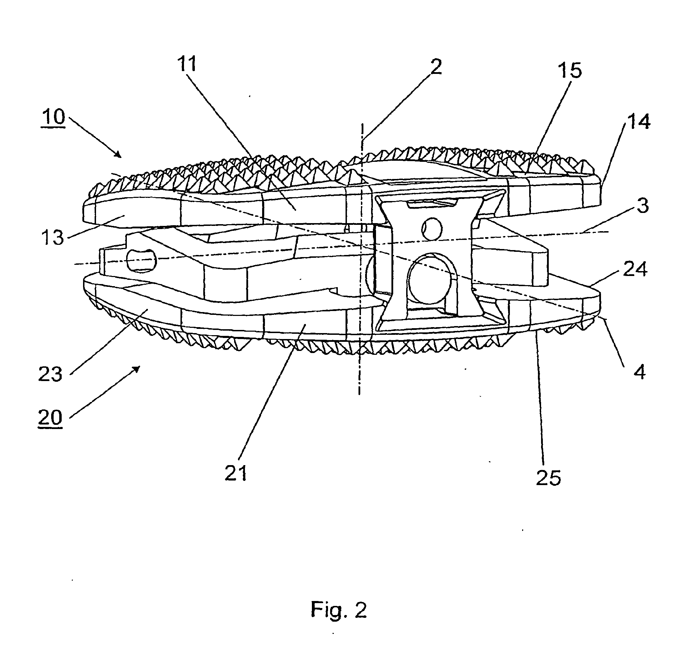

In den Fig. 1 und 2 ist eine Ausführungsform des erfindungsgemässen Zwischenwirbelimplantates 1 dargestellt, welche ein oberes Teil 10 mit einer oberen, quer zur Zentralachse 2 angeordneten Appositionsfläche 15 zur Anlage an die Grundplatte eines angrenzenden Wirbelkörpers, ein unteres Teil 20 mit einer unteren, quer zur Zentralachse 2 angeordneten Appositionsfläche 25 zur Anlage an die Deckplatte des angrenzenden Wirbelkörpers und zwei Gelenke 38;39 umfasst. Das obere Teil 10 und das untere Teil 20 sind über die Gelenke 38;39 relativ zueinander bewegbar verbunden, wobei die Bewegbarkeit des oberen Teils 10 relativ zum unteren Teil 20 um eine erste, quer zur Zentralachse 2 angeordnete Drehachse 3 innerhalb eines Winkelbereiches von +10° bis -6° eingeschränkt ist und um eine zweite, quer zur Zentralachse 2 und senkrecht zur ersten Drehachse 3 angeordneten Drehachse 4 innerhalb eines Winkelbereiches von ± 7° eingeschränkt ist

Die beiden Gelenke 38;39 sind durch drei Gelenkteile 31;32;33 realisiert, wovon das untere Gelenkteil 33 und das obere Gelenkteil 31 je ein mit dem mittleren Gelenkteil 32 zuammenwirkendes Gelenk 38;39 bilden. Die zwei Gelenke 38;39 weisen je eine Drehachse 3;4 auf, wobei die Drehachsen senkrecht aufeinander und senkrecht zur Zentralachse 2 stehen. Das untere Gelenk 39 umfasst als Artikulationsflächen eine am mittleren Gelenkteil 32 angeordnete zur ersten Drehachse 3 koaxiale, untere konvexe Gleitfläche 57 und eine am unteren Gelenkteil 33 angeordnete, zur Gleitfläche 5 komplementäre, untere konkave Gleitfläche 58. Das obere Gelenk 38 umfasst als Artikulationsflächen eine am oberen Gelenkteil 31 angeordnete zur zweiten Drehachse 4 koaxiale, obere konvexe Gleitfläche 55 und eine am mittleren Gelenkteil 32 angeordnete, zur Gleitfläche 55 komplementäre, untere konkave Gleitfläche 56 zusammen. Die Gleitflächen 55;56;57;58 sind als Teilflächen von Kreiszylindermantelflächen ausgestaltet.

Ferner sind am oberen und am mittleren Gelenkteil 31;32 axial endständig zu den Drehachsen 3;4 koaxiale Nocken 90 angebracht, welche in Langlochführungen 91 im unteren Gelenkteil und im mittleren Gelenkteil 32 verschiebbar aufgenommen sind. Durch die in den Langlochführungen 91 geführten Nocken 90 werden die Drehwinkel der Gelenkteile 31;32;33 um die Drehachsen 3;4 begrenzt. Zudem wird das Zwischenwirbelimplantat 1 durch die in den Langlochführungen 91 aufgenommenen Nocken 90 zusammengehalten.

Die Bewegbarkeit der beiden Teile 10;20 relativ zueinander ist durch die Mittel 40 lösbar blockierbar. Die Mittel 40 umfassen in der hier dargestellten Ausführungsform einen von den ventralen Seitenflächen 11;21 der beiden Teile 10;20 her quer zur Zentralachse 2 und parallel zu den lateralen Seitenflächen 13;14;23;24 der beiden Teile 10;20 einschiebbaren Einsatz 41. Das Einschieben des Einsatzes 41 erfolgt in zwei Vertiefungen 42;43, welche als Schwalbenschwanzführungen ausgestaltet sind. Der Einsatz 41 wird von den ventralen Seitenflächen 11;21 der beiden Teile 10;20 in die als Schwalbenschwanzführungen ausgestalteten Vertiefungen 42;43 eingeführt und am unteren Teil 20 mittels einer Schraube 44 befestigt. Zudem ist der Einsatz 41 endständig komplementär zu den Vertiefungen 42;43 ausgestaltet, so dass die beiden Teile 10;20 bei eingeschobenem Einsatz 41 parallel zur Zentralachse 2 relativ zueinander fixiert sind.

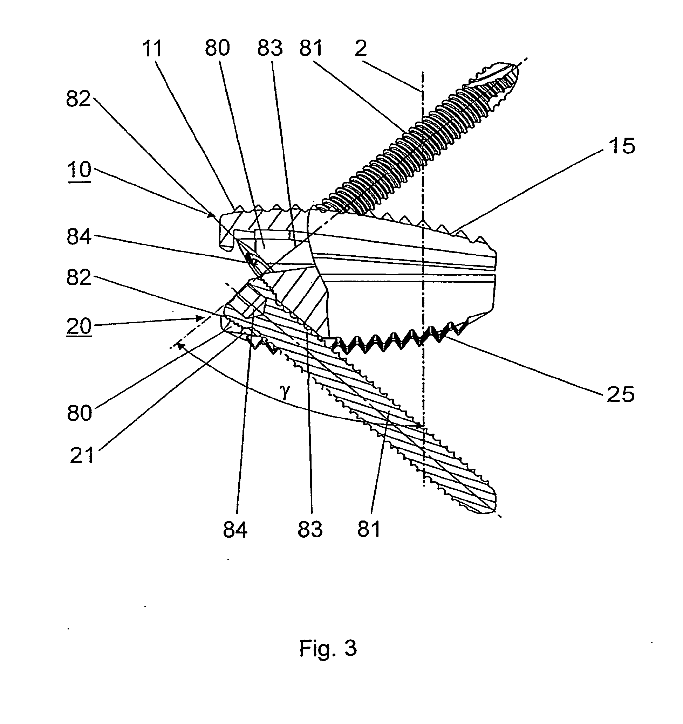

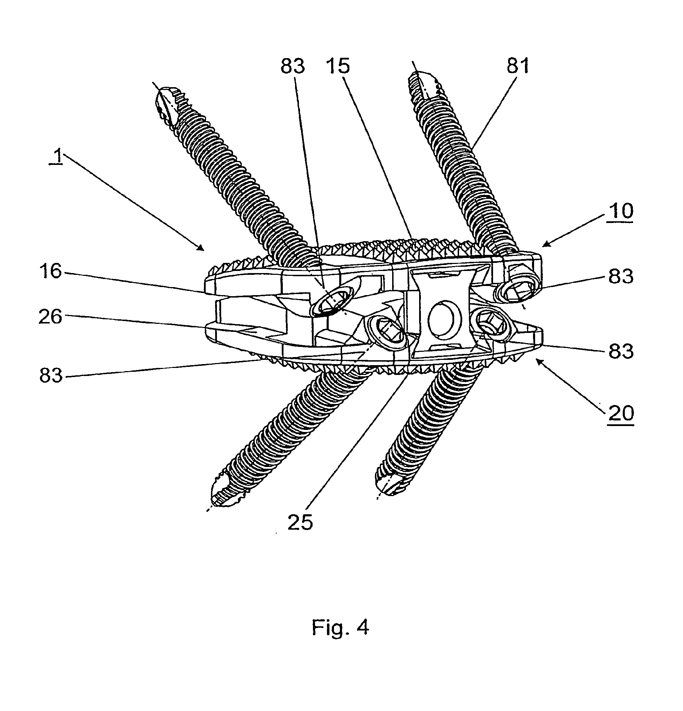

In Fig. 3 und 4 ist eine Ausführungsform des erfindungsgemässen Zwischenwirbelimplantates 1 dargestellt, welche sich von der in den Fig. 1 und 2 dargestellten Ausführungsform nur darin unterscheidet, dass die beiden Teilen 10;20 Bohrungen 80 zur Aufnahme von Knochenfixationsmitteln 81 umfassen, wobei die Knochenfixationsmittel 80 hier als Knochenschrauben ausgestaltet sind. Die Bohrungen 80 weisen Längsachsen 83 auf, welche einen Winkel γ mit der Zentralachse 2 einschliessen. Ferner durchdringen je zwei Bohrungen 80 eines der beiden Teile 10;20 von der ventralen Seitenfläche 11;21 zur Appositionsfläche 15;25. Die Längsachsen 83 der Bohrungen 80 stehen sowohl von lateral betrachtet (Fig. 3) als auch von ventral betrachtet (Fig. 4) schräg zur Zentralachse 2. Ferner sind die Bohrungen 80 konisch, sich gegen die Appositionsflächen 15;25 verjüngend ausgestaltet und mit Innengewinden 82 versehen, welche zur schraubbaren Aufnahme der mit komplementären Aussengewinden versehenen Schraubenköpfe 84 der als Knochenschrauben ausgestalteten Knochenfixationsmittel 81 dienen.

## Patentansprüche

1. Zwischenwirbelimplantat (1), insbesondere künstliche Bandscheibe, mit einer Zentralachse (2), einem oberen Teil (10), das für die Anlage an die Grundplatte eines darüber liegenden Wirbelkörpers geeignet ist und einem unteren Teil (20), das für die Anlage an die Deckplatte eines darunter liegenden Wirbelkörpers geeignet ist, wobei
A) das obere Teil (10) eine ventrale Seitenfläche (11), eine dorsale Seitenfläche (12), zwei laterale Seitenflächen (13,14), eine obere Appositionsfläche (15) und eine untere Oberfläche (16) aufweist;
B) das untere Teil (20) eine ventrale Seitenfläche (21), eine dorsale Seitenfläche (22), zwei laterale Seitenflächen (23,24), eine untere Appositionsfläche (25) und eine obere Oberfläche (26) aufweist; wobei
C) die beiden Teile (10;20) durch zwei zwischen den beiden Teilen (10;20) angeordnete Gelenke (38;39) relativ zueinander bewegbar sind;
D) jedes der Gelenke (38;39) eine Drehachse (3;4) aufweist und die beiden Drehachsen (3;4) quer zueinander angeordnet sind; und
E) die beiden Gelenke (38;39) durch ein mit dem oberen Teil (10) verbundenes, oberes Gelenkteil (31), ein mittleres Gelenkteil (32) und ein mit dem unteren Teil (20) verbundenes Gelenkteil (33) realisiert sind;
F) eines der aussenstehenden Gelenkteile (31;33) mindestens eine bezüglich einer Drehachse (3;4) rotationssymmetrische, konkave Gleitfläche (58) umfasst;
G) das mittlere Gelenkteil (32) mindestens eine zu dieser konkaven Gleitfläche (58) komplementäre, konvexe Gleitfläche (57) umfasst,
H) das andere der aussenstehenden Gelenkteile (31;33) mindestens eine bezüglich der anderen Drehachse (3;4) rotationssymmetrische, konvexe Gleitfläche (55) umfasst;
I) das mittlere Gelenkteil (32) mindestens eine zu dieser konvexen Gleitfläche (55) komplementäre, konkave Gleitfläche (56) umfasst; und
J) die Gleitflächen (55;56;57;58) als Teilflächen von Kreiszylinder- oder Kreiskegel-Mantelflächen ausgestaltet sind;
**dadurch gekennzeichnet, dass**
K) Mittel (40) vorgesehen sind, welche geeignet sind eine temporäre Blockierung der Beweglichkeit der beiden Teile (10,20) um die Gelenke (38;39) herbeizuführen.

2. Zwischenwirbelimplantat (1) nach Anspruch 1, **dadurch gekennzeichnet, dass** das untere Gelenkteil (33) mindestens eine bezüglich der ersten Drehachse (3) rotationssymmetrische, untere konkave Gleitfläche (58) umfasst und das mittlere Gelenkteil (32) mindestens eine zur unteren konkaven Gleitfläche (58) komplementäre, untere konvexe Gleitfläche (57) umfasst.

3. Zwischenwirbelimplantat (1) nach Anspruch 1 oder 2, **dadurch gekennzeichnet, dass** das obere Gelenkteil (31) mindestens eine bezüglich der zweiten Drehachse (4) rotationssymmetrische, obere konvexe Gleitfläche (55) umfasst und das mittlere Gelenkteil (32) mindestens eine zur oberen konvexen Gleitfläche (55) komplementäre, obere konkave Gleitfläche (56) umfasst.

4. Zwischenwirbelimplantat (1) nach einem der Ansprüche 1 bis 3, **dadurch gekennzeichnet, dass** Mittel (40) vorgesehen sind, welche die beiden Teile (10;20), bei ihren ventralen Seitenflächen (11;21) gemessen, auf einer festen Distanz voneinander halten.

5. Zwischenwirbelimplantat (1) nach einem der Ansprüche 1 bis 4, **dadurch gekennzeichnet, dass** die Drehachsen (3;4) windschief zueinander angeordnet sind.

6. Zwischenwirbelimplantat (1) nach einem der Ansprüche 1 bis 5, **dadurch gekennzeichnet, dass** die Mittel (40) an den beiden ventralen Seitenflächen (11,21) an den beiden Teilen (10;20) anbringbar sind.

7. Zwischenwirbelimplantat (1) nach einem der Ansprüche 1 bis 6, **dadurch gekennzeichnet, dass** die Mittel (40) einen Einsatz (41) mit einem unteren Ende (45) und einem oberen Ende (46) und an den beiden Teilen (10;20) je eine Vertiefung (42;43) in den Oberflächen (16;26) umfassen, welche an den ventralen Seitenflächen (11;21) offen sind, und dass der Einsatz (41) mit seinen Enden (45;46) in je eine Vertiefung (42;43) einfügbar ist.

8. Zwischenwirbelimplantat (1) nach Anspruch 7, **dadurch gekennzeichnet, dass** die Vertiefungen (42;43) Schwalbenschwanzführungen sind und die Enden (45;46) am Einsatz (41) komplementär zu diesen Schwalbenschwanzführungen ausgestaltet sind.

9. Zwischenwirbelimplantat (1) nach Anspruch 8, **dadurch gekennzeichnet, dass** sich die Schwalbenschwanzführungen von den ventralen Seitenflächen (11;21) her gegen die dorsalen Seitenflächen (12;22) verjüngen.

10. Zwischenwirbelimplantat (1) nach einem der Ansprüche 1 bis 9, **dadurch gekennzeichnet, dass** das obere und das untere Teil (10;20) je mindestens zwei von den ventralen Seitenflächen (11;21) zu den Appositionsflächen (15;25) durchgehende Bohrungen (80) mit Längsachsen (83) zur Aufnahme von Knochenfixationsmitteln (81) umfassen.

11. Zwischenwirbelimplantat (1) nach Anspruch 10, **dadurch gekennzeichnet, dass** die Längsachsen (83) der Bohrungen (80) mit der Zentralachse (2) einen Winkel γ einschliessen.

12. Zwischenwirbelimplantat (1) nach Anspruch 11, **dadurch gekennzeichnet, dass** der Winkel γ in einem Bereich von 20° und 65° liegt.

13. Zwischenwirbelimplantat (1) nach einem der Ansprüche 10 bis 12, **dadurch gekennzeichnet, dass** die Längsachsen (83) der Bohrungen (80) von den ventralen Seitenflächen (11;21) aus betrachtet von den inneren Oberflächen (16;26) gegen die Appositionsflächen (15;25) divergieren.

14. Zwischenwirbelimplantat (1) nach einem der Ansprüche 10 bis 13, **dadurch gekennzeichnet, dass** sich die Bohrungen (80) gegen die Appositionsflächen (15;25) konisch verjüngen.

15. Zwischenwirbelimplantat (1) nach einem der Ansprüche 10 bis 14, **dadurch gekennzeichnet, dass** die Bohrungen (80) ein Innengewinde (82) aufweisen.

## Claims

1. Intervertebral implant (1), specifically an artificial intervertebral disk, with a central axis (2), an upper section (10), suitable for laying onto the base plate of a vertebral body lying above and a lower section (20) suitable for laying onto the cover plate of a vertebral body lying below, wherein
A) the upper section (10) is provided with a ventral side area (11), a dorsal side area (12), two lateral side areas (13,14), a top apposition surface (15) and a bottom surface (16);
B) the lower section (20) is provided with a ventral side area (21), a dorsal side area (22), two lateral side areas (23,24), a bottom apposition surface (25) and a top surface (26); wherein
C) the two sections (10;20) are moveable in relation to each other by means of two joints (38;39) arranged between the two sections (10;20),
D) each of the joints (38;39) is provided with a swivel axis (3;4) and the two swivel axes (3;4) are arranged transversely to each other;
E) the two joints (38;39) are realised by means of an upper joint element (31) connected with the upper section (10), a central joint element (32) and a joint element (33) connected with the lower section (20);
F) one of the external joint sections (31;33) comprises at least one concave sliding surface (58) rotationally symmetrical with regard to a swivel axis (3;4);
G) the central joint section (32) comprises at least one convex sliding surface (57) complementary to this concave sliding surface (58);
H) the other of the external joint sections (31;33) comprises at least one convex sliding surface (55) rotationally symmetrical with regard to the other swivel axis (3;4);
I) the central joint section (32) comprises at least one concave sliding surface (56) complementary to this convex sliding surface (55); and
J) the sliding surfaces (55;56;57;58) are configured as partial surface areas of circular cylindrical or circular conical surface areas,
**characterised in that**
K) a means (40) is provided that is suitable for causing temporary blocking of the mobility of the two sections (10,20) around the joints (38;39).

2. Intervertebral implant (1) according to claim 1, **characterised in that** the lower joint element (33) comprises at least one lower concave sliding surface (58) rotationally symmetrical with regard to the first swivel axis (3) and the central joint section (32) comprises at least one lower convex sliding surface (57) complementary to the lower concave sliding surface (58),.

3. Intervertebral implant (1) according to claim 1 or claim 2, **characterised in that** the upper joint element (31) comprises at least one upper convex sliding surface (55) rotationally symmetrical with regard to the second swivel axis (4) and the central joint section (32) at least one upper concave sliding surface (56) complementary to the upper convex sliding surface (55).

4. Intervertebral implant (1) according to one of the claims 1 to 3, **characterised in that** a means (40) is provided that keeps the two sections (10;20), measured at their ventral side areas (11;21), at a fixed distance from each other.

5. Intervertebral implant (1) according to one of the claims 1 to 4, **characterised in that** the swivel axes (3;4) are arranged skewed to each other.

6. Intervertebral implant (1) according to claim 4 or claim 5, **characterised in that** the means (40) can be attached to the two ventral side areas (11,21) of the two sections (10;20).

7. Intervertebral implant (1) according to one of the claims 4 to 6, **characterised in that** the means (40) comprises an insert (41) with a lower end (45) and an upper end (46) and a depression (42;43) in the surfaces (16;26) at each of the two sections (10;20), which are open on the ventral side areas (11;21), and that the insert (41) with its ends (45;46) can be inserted into each of the depressions (42;43).

8. Intervertebral implant (1) according to claim 7, **characterised in that** the depressions (42;43) are dovetail guides and the ends (45;46) on the insert (41) are configrued complementary to these dovetail guides.

9. Intervertebral implant (1) according to claim 8, **characterised in that** the dovetail guides are tapered from the ventral side areas (11;21) towards the dorsal side areas (12;22).

10. Intervertebral implant (1) according to one of the claims 1 to 9, **characterised in that** the upper and the lower section (10;20) each comprises at least two bore holes (80) running through from the ventral side areas (11;21) to the apposition surfaces (15;25) with longitudinal axes (83) for receiving bone fixation means (81).

11. Intervertebral implant (1) according to claim 10, **characterised in that** the longitudinal axes (83) of the bore holes (80) enclose an angle γ with the central axis (2).

12. Intervertebral implant (1) according to claim 11, **characterised in that** the angle γ is in a range of between 20° and 65°.

13. Intervertebral implant (1) according to one of the claims 10 to 12, **characterised in that** the longitudinal axes (83) of the bore holes (80) as seen from the ventral side areas (11;21) diverge from the inner surfaces (16;26) against the apposition surfaces (15;25).

14. Intervertebral implant (1) according to one of the claims 10 to 13, **characterised in that** the bore holes (80) are conically tapered towards the apposition surfaces (15;25).

15. Intervertebral implant (1) according to one of the claims 10 to 14, **characterised in that** the bore holes (80) are provided with an internal thread (82).

## Revendications

1. Implant intervertébral (1), en particulier disque intervertébral artificiel, présentant un axe central (2), une partie supérieure (10), qui est appropriée à l'appui sur le plateau inférieur d'un corps vertébral sus-jacent, et une partie inférieure (20), qui est appropriée à l'appui sur un corps vertébral sous-jacent, dans lequel
A) la partie supérieure (10) présente une face latérale ventrale (11), une face latérale dorsale (12), deux faces latérales sur le côté (13, 14), une surface d'apposition supérieure (15) et une surface inférieure (16) ;
B) la partie inférieure (20) présente une face latérale ventrale (21), une face latérale dorsale (22), deux faces latérales sur le côté (23, 24), une surface d'apposition inférieure (25) et une surface supérieure (26) ; dans laquelle
C) les deux parties (10 ; 20) sont mobiles l'une par rapport à l'autre au moyen de deux articulations (38 ; 39) disposées entre les deux parties (10 ; 20) ;
D) chacune des articulations (38 ; 39) présente un axe de rotation (3 ; 4) et les deux axes de rotation (3 ; 4) sont disposés transversalement l'un à l'autre ; et
E) les deux articulations (38 ; 39) sont formées par une partie d'articulation supérieure (31) reliée à la partie supérieure (10), une partie d'articulation intermédiaire (32) et une partie d'articulation inférieure (33) reliée à la partie inférieure (20),
F) l'une des parties d'articulation externes (31 ; 33) comprend au moins une surface de glissement concave (58), avec une symétrie de rotation par rapport à un axe de rotation (3 ; 4) ;
G) la partie d'articulation intermédiaire (32) comprend au moins une surface de glissement convexe (57), complémentaire à cette surface de glissement concave (58),
H) l'autre des parties d'articulation externes (31 ; 33) comprend au moins une surface de glissement convexe (55), avec une symétrie de rotation par rapport à l'autre axe de rotation (3 ; 4) ;
I) la partie d'articulation intermédiaire (32) comprend au moins une surface de glissement concave (56), complémentaire à cette surface de glissement convexe (55) ; et
J) les surfaces de glissement (55 ; 56 ; 57 ; 58) sont réalisées en tant que surfaces partielles d'enveloppes externes en forme de cylindre circulaire ou de cône circulaire ; et
**caractérisé en ce que**
K) des moyens (40) sont prévus pour provoquer un blocage temporaire de la mobilité des deux parties (10, 20) autour de l'articulation (38 ; 39).

2. Implant intervertébral (1) selon la revendication 1, **caractérisé en ce que** la partie d'articulation inférieure (33) comprend au moins une surface de glissement inférieure concave (58), avec une symétrie de rotation par rapport au premier axe de rotation (3), et la partie d'articulation intermédiaire (32) comprend au moins une surface de glissement inférieure convexe (57), complémentaire à la surface de glissement inférieure concave (58).

3. Implant intervertébral (1) selon la revendication 1 ou 2, **caractérisé en ce que** la partie d'articulation supérieure (31) comprend au moins une surface de glissement supérieure convexe (55), avec une symétrie de rotation par rapport à un deuxième axe de rotation (4), et la partie d'articulation intermédiaire (32) comprend au moins une surface de glissement supérieure concave (56), complémentaire à la surface de glissement supérieure convexe (55).

4. Implant intervertébral (1) selon l'une quelconque des revendications 1 à 3, **caractérisé en ce que** des moyens (40) sont prévus pour maintenir les deux parties (10 ; 20) à distance fixe l'une de l'autre, mesuré au niveau de leurs faces latérales ventrales (11 ; 21).

5. Implant intervertébral (1) selon l'une quelconque des revendications 1 à 4, **caractérisé en ce que** les axes de rotation (3 ; 4) sont disposés de manière inclinée l'un par rapport à l'autre.

6. Implant intervertébral (1) selon l'une quelconque des revendications 1 à 5, **caractérisé en ce que** les moyens (40) peuvent être placés sur les deux faces latérales ventrales (11, 21) des deux parties (10 ; 20).

7. Implant intervertébral (1) selon l'une quelconque des revendications 1 à 6, **caractérisé en ce que** les moyens (40) comprennent une pièce rapportée (41) présentant une extrémité inférieure (45) et une extrémité supérieure (46) et, au niveau des deux parties (10 ; 20), un évidement (42 ; 43) dans les surfaces (16 ; 26) qui sont ouvertes au niveau des faces latérales ventrales (11 ; 21), et **en ce que** la pièce rapportée (41) peut être insérée avec ses extrémités (45 ; 46) respectivement dans un évidement (42 ; 43).

8. Implant intervertébral (1) selon la revendication 7, **caractérisé en ce que** les évidements (42 ; 43) sont des guidages en queue d'aronde et les extrémités (45 ; 46) sont conçues, au niveau de la pièce rapportée (41), de manière complémentaire à ces guidages en queue d'aronde.

9. Implant intervertébral (1) selon la revendication 8, **caractérisé en ce** les guidages en queue d'aronde rétrécissent en partant des faces latérales ventrales (11 ; 21) jusqu'aux faces latérales dorsales (12 ; 22).

10. Implant intervertébral (1) selon l'une quelconque des revendications 1 à 9, **caractérisé en ce que** les parties supérieure et inférieure (10 ; 20) comprennent chacune au moins deux alésages (80) présentant des axes longitudinaux (83), s'étendant des faces latérales ventrales (11 ; 21) aux surfaces d'apposition (15 ; 25) pour loger des moyens de fixation osseuse (81).

11. Implant intervertébral (1) selon la revendication 10, **caractérisé en ce que** les axes longitudinaux (83) des alésages (80) forment un angle γ avec l'axe central (2).

12. Implant intervertébral (1) selon la revendication 11, **caractérisé en ce que** l'angle γ se situe dans une gamme de 20° à 65°.

13. Implant intervertébral (1) selon l'une quelconque des revendications 10 à 12, **caractérisé en ce que** les axes longitudinaux (83) des alésages (80), vu depuis les faces latérales ventrales (11 ; 21), divergent depuis les surfaces internes (16 ; 26) jusqu'aux surfaces d'apposition (15 ; 25).

14. Implant intervertébral (1) selon l'une quelconque des revendications 10 à 13, **caractérisé en ce que** les alésages (80) rétrécissent de manière conique vers les surfaces d'apposition (15 ; 25).

15. Implant intervertébral (1) selon l'une quelconque des revendications 10 à 14, **caractérisé en ce que** les alésages (80) présentent un taraudage (82).
